# FASCICULE DE BREVET EUROPEEN

(11) **EP 0 922 748 B1**
(45) Date de publication et mention de la délivrance du brevet: **17.09.2003**
(21) Numéro de dépôt: 98402872.0
(22) Date de dépôt: 19.11.1998
(51) Int. Cl.: C10G 53/02, C10G 53/08

(54) **Procédé de séparation d'une charge C5-C8 ou d'une charge intermédiaire, en trois effluents respectivement riches en paraffines linéaires, monobranchées et multibranchées**
Verfahren zur Trennung von C5-C8 Einsätzen in drei Produkte, angereicht in lineare, einfach-verzweigte und vielfach-verzweigte Paraffine
Process for the separation of a C5-C8 feed in three fractions rich in normal , mono-branched and multi-branched paraffins

(30) Priorité: 25.11.1997 FR 9714888
(43) Date de publication de la demande: 16.06.1999
(73) Titulaire: Institut Français du Pétrole, 92500 Rueil Malmaison (FR)
(72) Inventeur: Ragil, Karine, 92500 Rueil Malmaison (FR); Prevost, Isabelle, 92500 Rueil Malmaison (FR); Clause, Olivier, 78400 Chatou (FR); LaRue, Joseph, 92500 Rueil Malmaison (FR); Millot, Benoit, 54410 Laneuveville Devant Nancy (FR)

(56) Documents cités:
- EP-A- 0 384 540
- EP-A- 0 473 828
- WO-A-93/19840
- US-A- 4 717 784

## Description

L'invention concerne un procédé de séparation permettant d'obtenir trois effluents respectivement riches en paraffines linéaires, en paraffines monobranchées et en paraffines dibranchées, tribranchées et éventuellement en composés naphténiques et/ou aromatiques, à partir de coupes C5-C8 ou de coupes intermédiaires (C5-C7, C6-C8 ou C6-C7), comprenant des hydrocarbures paraffiniques, naphténiques, aromatiques et oléfiniques. Le procédé de séparation selon l'invention met en oeuvre au moins deux unités de séparation fonctionnant soit par adsorption soit par perméation. Le procédé peut aussi résulter de la combinaison de ces deux techniques de séparation. Le procédé est adapté à un fonctionnement en phase liquide ou en phase gazeuse. Dans le cas où la séparation utilise au moins une unité d'adsorption, la séparation peut être réalisée à partir d'adsorbants capables d'adsorber préférentiellement les paraffines linéaires, ou à partir d'adsorbants capables d'adsorber préférentiellement les paraffines monobranchées. Dans le cas où la séparation est effectuée par perméation, la séparation de l'isomérat peut être réalisée en utilisant une technique de perméation gazeuse ou de pervaporation. Le procédé de séparation selon l'invention est particulièrement utile lorsqu'il est couplé avec le procédé d'hydro-isomérisation tel que décrit dans la demande de brevet ayant pour titre : « Essences à haut indice d'octane, et leur production par un procédé combinant hydro-isomérisation et séparation », déposée le même jour par la Demanderesse, puisqu'il permet un recyclage sélectif des paraffines linéaires et monobranchées, nécessaire pour les paraffines comportant au moins 7 atomes de carbone.

Dans le cas où la charge du procédé comprend la coupe C5, de l'isopentane issu de cette coupe est retiré des flux traversant le procédé à l'aide d'au moins un déisopentaniseur disposé en amont et/ou en aval des différentes unités de séparation pour servir d'éluant ou de gaz de balayage, respectivement dans les unités de séparation par adsorption ou par perméation.

### ART ANTÉRIEUR

La prise en compte de contraintes environnementales accrues entraîne la suppression des composés du plomb dans les essences, effective aux Etats-Unis et au Japon et en voie de généralisation en Europe. Dans un premier temps, les composés aromatiques, constituants principaux des essences de réformage, les isoparaffines produites par alkylation aliphatique ou isomérisation d'essences légères ont compensé la perte d'indice d'octane résultant de la suppression du plomb dans les essences. Par la suite, des composés oxygénés tels que le Méthyl Tertiobutyl Ether (MTBE) ou l'Ethyl Tertiobutyl Ether (ETBE) ont été introduits dans les carburants. Plus récemment, la toxicité reconnue de composés tels que les aromatiques, en particulier le benzène, les oléfines et les composés soufrés, ainsi que la volonté de diminuer la pression de vapeur des essences, ont entraîné aux Etats-Unis la production d'essences reformulées. Par exemple, les teneurs maximales en oléfines, composés aromatiques totaux et benzène dans les essences distribuées en Californie en 1996 sont respectivement de 6 % vol., 25 % vol. et 1% vol. En Europe, les spécifications sont moins sévères ; néanmoins, la tendance prévisible est une réduction semblable des teneurs maximales en benzène, en composés aromatiques et en oléfines dans les essences produites et vendues.

Les «pools essence» comprennent plusieurs composants. Les composants majoritaires sont l'essence de réformage, qui comprend habituellement entre 60 et 80 % vol. de composés aromatiques, et les essences de craquage catalytique (FCC), qui contiennent typiquement 35 % vol. d'aromatiques mais apportent la majorité des composés oléfiniques et soufrés présents dans les essences. Les autres composants peuvent être les alkylats, sans composés aromatiques ni oléfiniques, les essences légères isomérisées ou non isomérisées, qui ne contiennent pas de composés insaturés, les composés oxygénés tels que le MTBE et des butanes. Dans la mesure où les teneurs maximales en aromatiques ne sont pas réduites en dessous de 30 ou 40 % vol., la contribution des réformats dans les «pools essence» restera importante, typiquement 40 % vol. Une sévérisation accrue de la teneur maximale admissible en composés aromatiques à 20 ou 25 % vol. entraînera une diminution de l'utilisation du réformage, et par voie de conséquence la nécessité de valoriser les coupes C7-C10 de distillation directe par d'autres voies que le réformage. Leur valorisation par hydro-isomérisation est une des voies possibles, comme décrit dans la demande de brevet ayant pour titre : « Essences à haut indice d'octane, et leur production par un procédé combinant hydro-isomérisation et séparation », déposée le même jour par la Demanderesse. Le procédé d'hydro-isomérisation conduit à la formation de composés multibranchés à partir des composés de faibles indices d'octane. Il ne peut être mis en oeuvre qu'à la condition de recycler les paraffines linéaires et monobranchées en C7-C10, puisque la réaction d'hydro-isomérisation est équilibrée et que ces paraffines de faibles indices d'octane ne peuvent pas être envoyées vers le «pool essence». De plus, des conditions d'hydroisomérisation différentes doivent être mises en oeuvre pour ces paraffines isomères afin d'éviter le craquage des paraffines les plus ramifiées. Ces deux points justifient la recherche de procédés de séparation permettant d'obtenir trois effluents distincts, respectivement un effluent riche en paraffines linéaires, un effluent riche en paraffines monobranchées et un effluent riche en paraffines multibranchées et éventuellement en composés naphténiques et/ou aromatiques.

L'utilisation de procédés de séparation par adsorption ou par perméation pour effectuer la séparation des paraffines linéaires, monobranchées et multibranchées a déjà fait l'objet de plusieurs brevets (par exemple US-A-4 717 784, 4 956 521 et 5 233 120, BE-A-891 522, FR-A-2 688 213, US-A-5 055 633, 4 367 364 et 4 517 402). Cependant, ces brevets ne s'appliquent d'une part qu'à la fraction légère C5-C6 et ne concernent de plus que la séparation de ces coupes de distillation en deux effluents, l'un de faible indice d'octane et l'autre de fort indice d'octane.

De même, les brevets US-A-4 210 771 et 4 709 116 décrivent la séparation des paraffines linéaires à partir d'une coupe naphta C5-C6 en utilisant un adsorbant connu dans l'industrie sous le nom de zéolithe 5A au calcium. De même, le brevet US-A-4 367 364 décrit cette même séparation effectuée à l'aide de silicalite (US-A-4 061 724). Les procédés de séparation décrits par ces brevets sont souvent couplés à un procédé d'isomérisation des paraffines linéaires puisque celles-ci possèdent un faible indice d'octane.

De la même façon, certains brevets (comme US-A-4717784 et 5 055 633) décrivent des procédés permettant d'effectuer la séparation des paraffines linéaires et des paraffines monobranchées à partir d'une coupe C5-C6. Ces paraffines linéaires et monobranchées constituent le pool de bas indice d'octane, alors que les paraffines multibranchées constituent le pool à haut indice d'octane. Dans ce cas, ces brevets soulignent l'intérêt d'utiliser des adsorbants tels que la ferriérite (US-A-4 804 802 et 4 717 784), les zéolithes ZSM-5 (US-A-3 702 886), ZSM-11 (US-A-4 108 881), ZSM-23 (US-A-4 076 842) et ZSM-35 (US-A-4 016 245) et la silicalite (US-A-5 055 633), puisque ces adsorbants adsorbent à la fois les paraffines linéaires et les paraffines monobranchées des coupes C5-C6 et excluent les paraffines de degrés de ramification plus élevés. Lors de la mise en oeuvre de tels adsorbants, l'isopentane est séparé de la charge et est produit dans le pool de faible indice d'octane, avec les paraffines linéaires et monobranchées, alors que ce composé possède un fort indice d'octane. Le brevet US-A-5 055 633 souligne en conséquence l'intérêt d'une mise en oeuvre permettant de produire l'isopentane avec le flux riche en composés multibranchés, composés naphténiques et/ou aromatiques à partir d'une charge C5-C6. Celle-ci contient au moins 10 % molaire d'isopentane, ainsi que des composés C7+ en quantités inférieures à 10 % molaire. Un tel procédé conduit à un flux secondaire riche en paraffines linéaires et monobranchées qui peut être envoyé vers un réacteur d'isomérisation.

Ces brevets n'envisagent pas d'effectuer le fractionnement en trois effluents des coupes C5-C6 dans le cadre de leur isomérisation pour deux raisons : d'une part, l'indice d'octane des paraffines monobranchées en C5-C6 est souvent jugé suffisant pour que ces composés soient envoyés vers le pool essence, auquel cas ces paraffines sont séparées avec les paraffines multibranchées. D'autre part, lorsque les paraffines linéaires et les paraffines monobranchées sont recyclées vers l'isomérisation, il n'est pas utile de les séparer, puisque ces composés peuvent être isomérisés dans les mêmes conditions opératoires, contrairement aux coupes plus lourdes telles que celles concernées par la présente invention. Le brevet US-A-5 055 634 est le seul à décrire un procédé pouvant conduire à trois flux respectivement riches en paraffines linéaires, monobranchées et multibranchées à partir d'une coupe légère C5-C6, mais son l'intérêt principal, tout comme celui du procédé du brevet US-A-5 055 633, réside dans la possibilité de séparer et de produire l'isopentane avec le flux riche en paraffines multibranchées. La charge d'un tel procédé contient au moins 10% d'isopentane. Elle est centrée sur C5-C6 et peut parfois contenir de faibles quantités de paraffines comportant sept atomes de carbone ou plus. En conséquence, le procédé décrit dans ce brevet s'applique pour des teneurs en ces composés C7+ inférieures à 10% molaire. Ce procédé comprend la mise en oeuvre de deux unités disposées en série. La charge arrive dans la première unité qui contient un adsorbant capable de retenir sélectivement les paraffines linéaires. L'effluent de cette unité est en conséquence constitué de paraffines mono- et multibranchées. Cet effluent dénormalisé est alors introduit dans la seconde unité remplie d'un adsorbant capable de retenir préférentiellement les paraffines monobranchées à l'exception de l'isopentane, qui est produit avec les paraffines multibranchées. Ce brevet indique que la régénération des deux unités s'effectue à l'aide d'un gaz non adsorbable comme l'hydrogène par exemple. Celui-ci parcourt tout d'abord la seconde unité et permet de désorber les paraffines monobranchées. Au moins une partie de ce flux est alors envoyé vers la première unité et permet de désorber les paraffines linéaires qui y sont contenues. La régénération ainsi effectuée conduit à mélanger une partie des paraffines monobranchées aux paraffines linéaires précédemment séparées à l'exception de l'isopentane, qui est récupéré avec les composés de forts indices d'octane dans le flux de production. Dans une version préférée de ce procédé, l'ensemble du flux de désorption sortant de la deuxième unité parcourt le premier afin de minimiser la quantité de gaz non adsorbable nécessaire pour régénérer l'ensemble dès deux unités. Dans ce dernier cas, le procédé ne produit que deux flux, le premier riche en paraffines multibranchées, composés naphténiques, aromatiques et isopentane, le second riche en paraffines linéaires et monobranchées. Une telle séparation peut alors être effectuée à l'aide d'un seul adsorbeur contenant deux types d'adsorbants conformément à l'exemple donné dans ce brevet.

Les techniques de séparation par adsorption utilisées par ces différents brevets afin de valoriser les coupes C5-C6 sont celles connues par l'homme de l'art. Ainsi, le procédé de séparation par adsorption peut être de type PSA (Pressure Swing Adsorption), TSA (Temperature Swing Adsorption), chromatographique (chromatographie d'élution ou contre-courant simulé par exemple) ou résulter d'une combinaison de ces techniques. Ces procédés ont tous en commun de mettre en contact un mélange liquide ou gazeux avec un lit fixe d'adsorbant afin d'éliminer certains constituants du mélange qui peuvent être adsorbés. La désorption peut être réalisée par différents moyens. Ainsi, la caractéristique commune de la famille des PSA est d'effectuer la régénération du lit par dépressurisation et dans certains cas par balayage à basse pression. Les procédés de type PSA sont décrits dans le brevet US-A-3 430 418 ou dans l'ouvrage plus général de Yang (« Gas Separation by Adsorption Processes », Butterworth Publishers, US, 1987). Des cycles basés sur l'utilisation de différents agencements de lits y sont en particulier décrits en détail. En général, les procédés de type PSA sont opérés de façon séquentielle et en utilisant alternativement tous les lits d'adsorption. Ces PSA ont remporté de nombreux succès dans le domaine du gaz naturel, de la séparation des composés de l'air, de la production de solvant et dans différents secteurs du raffinage.

Les procédés TSA, qui utilisent la température comme force motrice de désorption, sont les premiers à avoir été développés en adsorption. Le chauffage du lit à régénérer est assuré par une circulation de gaz préchauffé, en boucle ouverte ou fermée, en sens inverse de celui de l'étape d'adsorption. De nombreuses variantes de schémas (voir également : « Gas Separation by Adsorption Processes », Butterworth Publishers, US, 1987) sont utilisées en fonction des contraintes locales et de la nature du gaz employé. Cette technique de mise en oeuvre est généralement utilisée dans les procédés de purification (séchage, désulfuration de gaz et liquides, purification du gaz naturel (US-A-4 770 676)).

La chromatographie, en phase gazeuse ou en phase liquide est une technique de séparation très efficace grâce à la mise en oeuvre d'un très grand nombre d'étages théoriques. Elle permet ainsi de tirer parti de sélectivités d'adsorption relativement faibles et de réaliser des séparations difficiles. Les procédés N-ISELF® de Elf Aquitaine (BE-A-891 522) pour la séparation des n/isoparaffines et ASAHI (Seko M., Miyake J., Inada K., Ind. Eng. Chem. Prod. Res. Develop.,1979, 18, 263) pour la séparation du paraxylène et de l'éthylbenzène d'une coupe C8 aromatique présentent ce type de mise en oeuvre. Ces procédés sont fortement concurrencés par les procédés continus à lit mobile simulé ou contre courant simulé. Ces derniers ont connus un très fort développement dans le domaine pétrolier (US-A-3 636 121 et 3 997 620). La régénération de l'adsorbant fait appel à la technique de déplacement par un désorbant, qui doit pouvoir être séparé par distillation de l'extrait et du raffinat.

Les techniques de séparation par perméation présentent l'avantage par rapport aux séparations par adsorption d'être continues et par conséquent d'être relativement simples de mise en oeuvre. De plus, elles sont reconnues pour leur modularité et leur compacité. Elles ont trouvé depuis une dizaine d'années leur place aux côtés des techniques d'adsorption en séparation de gaz, par exemple pour récupérer l'hydrogène des gaz de raffinerie, décarbonater le gaz naturel, produire de l'azote d'inertage (« Handbook of Industrial Membranes », Elsevier Science Publishers, UK, 1995). Leur utilisation pour séparer des hydrocarbures isomères est rendue possible grâce aux progrès récents des techniques de synthèse de matériaux inorganiques permettant de faire croître des cristaux de zéolithe sous forme de couche mince continue supportée ou auto-supportée.

La demande internationale WO-A-96/01 687 décrit une méthode de synthèse de membrane zéolithe supportée et ses applications notamment en vue de la séparation d'un mélange de normal et d'isopentane. Une autre méthode de synthèse de membrane zéolithe supportée adaptée en particulier à la séparation des alcanes linéaires d'un mélange d'hydrocarbures plus branchés est décrite dans la demande internationale WO-A-93/19 840.

Des mesures de perméabilité d'hydrocarbures linéaires et branchés sur des films de zéolithe auto-supportés ou déposés sur des supports de différentes natures ont été rapportés dans la littérature. Par exemple Tsikoyiannis J.G. et Haag W.O., Zeolithe 1992,12,126-30, observent sur un film auto-supporté de ZSM-5, un rapport de perméabilité de 17,2 pour le nC6 par rapport à iC6.

Des mesures de perméabilités en gaz purs sur une membrane composée de cristaux de silicalite sur un support en acier poreux montre que le flux de nC4 est supérieur à celui de l'iC4 (Geus E.R., Van Bekkum H., Bakker W.J.W., Moulijn J.A., Microporous Mater.,1993,1,1311-47). Pour ces mêmes gaz, le rapport des perméabilités (nC4 / iC4) est de 18, à 30°C, et de 31, à 185°C, avec une membrane de zéolithe ZSM-5 sur support poreux en alumine. En ce qui concerne la séparation nC6/2,2 diméthylbutane une sélectivité de 122 a pu être mesurée avec des membranes de silicalite sur support en verre poreux (Meriaudeau P., Thangaraj A. et Naccache C., Micro porous Mater., 1995, 4, 213-219).

On connaît enfin le document EP-A-0 384 540, qui décrit notamment un procédé de séparation de charges d'hydrocarbures, particulièrement en C6+, contenant des paraffines linéaires, des paraffines monométhyl-branchées et des paraffines diméthyl-branchées, procédé qui comprend :
- le passage de ladite charge edans une première zone de séparation où sont séparés un flux contenant les paraffines linéaires et un flux contenant les paraffines monométhyl-branchées et les paraffines diméthyl-branchées ; et
- le passage de ce dernier fluxdans une seconde zone de séparation, où sont séparées les paraffines monométhyl-branchées et les paraffines diméthyl-branchées.

### RÉSUMÉ DE L'INVENTION

L'invention concerne un procédé de séparation permettant d'obtenir trois effluents respectivement riches en paraffines linéaires, en paraffines monobranchées et en paraffines dibranchées, tribranchées et éventuellement en composés naphténiques et/ou aromatiques, à partir de coupes légères C5-C8 ou de coupes intermédiaires, telles que C5-C7, C6-C8, ou C6-C7, comprenant des hydrocarbures paraffiniques et éventuellement naphténiques, aromatiques et/ou oléfiniques. Le procédé de séparation selon l'invention met en oeuvre au moins deux unités de séparation disposées en série fonctionnant soit par adsorption, soit par perméation (utilisant une ou plusieurs membranes). Le procédé peut aussi résulter de la combinaison de ces techniques de séparation. Dans ce procédé, de l'isopentane ou de l'isohexane contenu dans la charge est séparé de celle-ci et sert d'éluant pour la régénération de la (ou des) unités de séparation par adsorption et/ou de gaz de balayage pour la régénération de la (ou des) unités de séparation par perméation. Le procédé selon l'invention est adapté à un fonctionnement en phase liquide ou en phase gazeuse. Un tel procédé de séparation est particulièrement utile lorsqu'il est couplé avec un procédé d'hydro-isomérisation tel que décrit dans la demande de brevet ayant pour titre : « Essences à haut indice d'octane et leur production par un procédé combinant hydro-isomérisation et séparation », déposée le même jour par la Demanderesse. En effet, le procédé décrit nécessite de recycler à la fois les paraffines linéaires (nCx, x = 5 à 8) et les paraffines monobranchées (monoC₍ₓ₋₁₎), puisque les paraffines linéaires et monobranchées en C7-C8 ont de faibles indices d'octane (voir Tableau 1 ci-dessous). De plus, des conditions d'hydro-isomérisation différentes doivent être mises en oeuvre pour ces deux types d'isomères pour éviter le craquage des paraffines les plus ramifiées. Ces deux points justifient la recherche de procédés de séparation permettant d'obtenir trois effluents distincts respectivement riches en paraffines linéaires nCx, en paraffines monobranchées monoC(ₓ₋₁) et en paraffines multibranchées (diC₍ₓ₋₂₎ ou triC₍ₓ₋₃₎), composés naphténiques et/ou aromatiques.

**Tableau 1**

| | | | | | | | | |
|---|---|---|---|---|---|---|---|---|
| Paraffine | nC7 | monoC6 | diC5 | tri C4 | nC8 | monoC7 | di C6 | tri C5 |
| RON | 0 | 42-52 | 80-93 | 112 | < 0 | 21-27 | 55-76 | 100-109 |
| MON | 0 | 23-39 | 84-95 | 101 | < 0 | 23-39 | 56-82 | 96-100 |

Dans une première version du procédé, une première unité de séparation permet de séparer les paraffines linéaires des paraffines ramifiées. Cette unité produit une charge dénormalisée qui est envoyée vers une seconde unité de séparation, qui permet de séparer, d'une part, les paraffines monobranchées et d'autre part, les paraffines multibranchées et les composés naphténiques et/ou aromatiques.

Dans une seconde version du procédé, la première unité permet de séparer les paraffines multibranchées et les composés naphténiques et/ou aromatiques des paraffines linéaires et monobranchées. Ces dernières sont envoyées vers la seconde unité de séparation qui effectue la séparation en deux effluents, l'un riche en paraffines monobranchées, l'autre riche en paraffines linéaires. Les régénérations des unités, lorsque celles-ci utilisent un ou plusieurs adsorbants, sont toujours indépendantes dans le sens ou elles ne contribuent pas à mélanger les différents isomères ainsi séparés. Dans le cas ou la charge du procédé comprend la coupe C5, de l'isopentane issu de cette coupe est retiré des flux traversant le procédé à l'aide d'un déisopentaniseur disposé en amont ou en aval des différentes unités de séparation pour servir d'éluant ou de gaz de balayage respectivement pour les procédés de séparation par adsorption ou par perméation.

### DESCRIPTION DÉTAILLÉE DE L'INVENTION

La charge traitée dans le procédé selon l'invention provient de la coupe C5-C8 ou de toutes coupes intermédiaires (comme par exemple C5-C7, C6-C8 ou C6-C7) issues de la distillation atmosphérique, d'une unité de réformage (réformat léger) ou d'une unité de conversion (naphta d'hydrocracking par exemple). Dans la suite du texte, cet ensemble de charge possible sera désigné par les termes « coupe C5-C8 et coupes intermédiaires ».

Elle est composée principalement de paraffines linéaires, monobranchées et multibranchées, de composés naphténiques, tels que les diméthylcyclopentanes, de composés aromatiques tels que le benzène ou le toluène et éventuellement de composés oléfiniques. On regroupera sous le vocable "paraffines multibranchées", toutes les paraffines dont le degré de branchement est égal ou supérieur à deux.

La charge peut notamment contenir du normal pentane, du 2-méthylbutane, du néopentane, du normal hexane, du 2-méthylpentane, du 3-méthylpentane, du 2,2-diméthylbutane, du 2,3 diméthylbutane, du normal heptane, du 2-méthylhexane, du 3-méthylhexane, du 2,2-diméthylpentane, du 3,3-diméthylpentane, du 2,3-diméthylpentane, du 2,4-diméthylpentane, du 2,2,3-triméthylbutane, du normal octane, du 2-méthylheptane, du 3-méthylheptane, du 4-méthylheptane, du 2,2-diméthylhexane, du 3,3-diméthylhexane, du 2,3-diméthylhexane, du 2,2-diméthylhexane, du 2,4-diméthylhexane, du 2,5-diméthylhexane, du 2,2,3-triméthylpentane, du 2,3,3-triméthylpentane, du 2,3,4-triméthylpentane. Dans la mesure où la charge vient des coupes C5-C8 ou de coupes intermédiaires (comme par exemple C5-C7, C6-C8 ou C6-C7) obtenues après distillation atmosphérique, elle peut de plus contenir des alcanes cycliques, tels que les diméthylcyciopentanes, des hydrocarbures aromatiques (tels que benzène, toluène, xylènes) ainsi que d'autres hydrocarbures C9+ (c'est à dire des hydrocarbures contenant au moins 9 atomes de carbones) en quantité moindre. Les charges C5-C8 ou celles composées de coupes intermédiaires d'origine réformat peuvent de plus contenir des hydrocarbures oléfiniques, en particulier lorsque les unités reforming sont opérées à basse pression.

La teneur en paraffines (P) dépend essentiellement de l'origine de la charge, c'est à dire de son caractère paraffinique ou naphténique et aromatique, parfois mesuré par le paramètre N+A (somme de la teneur en naphtènes (N) et de la teneur en aromatiques (A)), ainsi que de son point initial de distillation, c'est-à-dire de la teneur en C5 et C6 dans la charge. Dans les naphtas d'hydrocracking, riches en composés naphténiques, ou les réformats légers, riches en composés aromatiques, la teneur en paraffines dans la charge sera en général faible, de l'ordre de 30 % en masse. Dans les coupes C5-C8 ou dans les coupes intermédiaires (comme par exemple C5-C7, C6-C8 ou C6-C7) de distillation directe, la teneur en paraffines varie entre 30 et 80 % en masse, avec une valeur moyenne de 55-60 % en masse.

La charge riche en paraffines comprenant entre 5 et 8 atomes de carbone est en général de faible indice d'octane et le procédé selon l'invention consiste à la fractionner en trois effluents distincts d'indices d'octane moteur et recherche croissants, respectivement riches en paraffines linéaires, en paraffines monobranchées et en paraffines dibranchées, tribranchées et éventuellement en composés naphténiques et/ou aromatiques.

Pour ce faire, au minimum deux unités de séparation sont à considérer. Plusieurs versions du procédé sont possibles suivant l'agencement des différentes unités.

Pour chacune des versions du procédé de cette invention, les séparations peuvent être effectuées en phase liquide ou gazeuse au moyen de procédés mettant en oeuvre des adsorbants ou des membranes. Les procédés de séparation par adsorption utilisés peuvent être du type PSA (Pressure Swing Adsorption), TSA (Temperature Swing Adsorption), chromatographique (chromatographie d'élution ou contre courant simulé par exemple) ou résulter d'une combinaison de ces mises en oeuvre. Les unités de séparation peuvent utiliser un ou plusieurs tamis moléculaires. De plus, généralement plusieurs unités de séparation (de deux à dix) sont utilisées en parallèle et alternativement pour conduire à un procédé fonctionnant de façon continue, alors que par nature les procédés d'adsorption sont discontinus. Dans le cas où la séparation est effectuée par perméation, la séparation de l'isomérat peut être réalisée en utilisant une technique de perméation gazeuse ou de pervaporation.

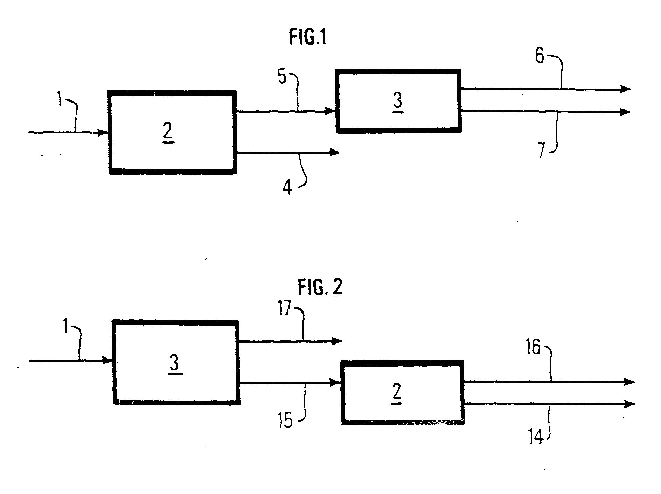

La version 1 du procédé est illustrée par la figure 1. La charge fraîche (flux 1) contenant des paraffines linéaires, monobranchées et multibranchées, composés naphténiques et/ou aromatiques arrive à l'unité de séparation 2. Les paraffines normales (flux 4) issues des coupes C5-C8 ou des coupes intermédiaires (C5-C7, C6-C8 ou C6-C7, sont séparées de la charge dans cette unité 2. Les caractéristiques concernant les adsorbants et les membranes capables d'effectuer une telle séparation sont données dans la suite du texte. L'unité 2 fournit à l'unité 3 une charge dénormalisée 5. Les paraffines monobranchées (flux 6) de cette charge vont être séparées de la charge dénormalisée 5 dans l'unité 3. Les caractéristiques concernant les adsorbants et les membranes capables d'effectuer une telle séparation sont données dans la suite du texte. L'unité 3 produit deux effluents, l'un riche en paraffines multibranchées, composés naphténiques et/ou aromatiques (flux 7), l'autre riche en paraffines monobranchées (flux 6). Ce procédé permet donc de fractionner en trois effluents 4, 6 et 7 d'indices d'octane et moteur croissants, une charge C5-C8 ou toutes charges intermédiaires.

La version 2 du procédé est illustrée par la figure 2. La charge fraîche (flux 1) contenant des paraffines linéaires, monobranchées et dibranchées, naphtènes et composés aromatiques arrive à l'unité de séparation 3. Les paraffines multibranchées, les composés naphténiques et/ou aromatiques (flux 17) issues des coupes C5-C8 ou des coupes intermédiaires (C5-C7, C6-C8 ou C6-C7) sont séparées de la charge dans cette unité 3. Les caractéristiques concernant les adsorbants et les membranes capables d'effectuer une telle séparation sont données dans la suite du texte. L'unité 3 fournit à l'unité 2 une charge de bas indice d'octane 15 contenant essentiellement des paraffines linéaires et monobranchées. Les paraffines monobranchées (flux 16) de cette charge vont être séparées de la charge 15 dans l'unité 2. Les caractéristiques concernant les adsorbants et les membranes capables d'effectuer une telle séparation sont données dans la suite du texte. L'unité 2 produit deux effluents, l'un riche en paraffines linéaires (flux 14), l'autre riche en paraffines monobranchées (flux 16). Ce procédé permet donc de fractionner en trois effluents 14, 16 et 17 d'indices d'octane et moteur croissants, une charge C5-C8 ou toutes charges intermédiaires.

Pour chacune de ces deux versions, lorsque la charge 1 contient la coupe C5, de l'isopentane provenant de cette coupe est retiré des flux traversant le procédé à l'aide d'au moins un déisopentaniseur disposé en amont et/ou en aval des différentes unités de séparation pour servir d'éluant ou de gaz de balayage respectivement dans les unités de séparation par adsorption ou par perméation.

Le procédé comprend au moins deux unités pouvant fonctionner chacune à l'aide d'adsorbant ou de membrane. Le procédé peut résulter de l'association d'au moins une unité fonctionnant par adsorption dans le but d'effectuer l'une des séparations et d'au moins une unité membranaire permettant d'effectuer l'autre séparation conformément à l'invention.

Lorsque au moins une des unités fonctionne par adsorption, elle est remplie d'un adsorbant naturel ou synthétique capable de séparer soit les paraffines linéaires des paraffines monobranchées, multibranchées, composés naphténiques et/ou aromatiques (version 1 unité 2), soit ces mêmes paraffines linéaires des paraffines monobranchées (version 2 unité 2), soit les paraffines multibranchées, les composés naphténiques et/ou aromatiques des paraffines monobranchées (version 1 unité 3), soit ces mêmes composés des paraffines monobranchées et linéaires (version 2 unité 3). La séparation à l'aide de tels adsorbants s'effectue sur la base des différences entre les propriétés géométriques, diffusionnelles ou thermodynamiques des adsorbats pour les adsorbants considérés. Il existe un grand nombre de matériaux adsorbants permettant d'effectuer ce type de séparation. Parmi eux, se trouvent les tamis moléculaires au carbone, les argiles activées, le silica gel, l'alumine activée et les tamis moléculaires cristallins. Ces derniers ont une taille de pore uniforme et sont pour cette raison particulièrement adaptés aux deux types de séparation. Ces tamis moléculaires incluent notamment les différentes formes de silicoaluminophosphates et d'aluminophosphates décrits dans les brevets US-A-4 444 871, 4 310 440 et 4 567 027 aussi bien que les tamis moléculaires zéolithiques. Ceux-ci sous leur forme calcinée peuvent être représentés par la formule chimique :

M_{2/n} O : Al₂O₃: x SiO₂: y H₂O

où M est un cation, x est compris entre 2 et l'infini, y a une valeur comprise entre 2 et 10 et n est la valence du cation. Dans le cadre de la séparation des paraffines linéaires (flux 4) de la charge 1 (unité 2, version 1), ou de ces mêmes paraffines linéaires (flux 14) des paraffines monobranchées (flux 16) (unité 2, version 2), des adsorbants dont la taille de pores est suffisante pour permettre l'adsorption des paraffines linéaires et exclure les molécules de tailles plus importantes telles que les paraffines monobranchées, multibranchées et les composés naphténiques et/ou aromatiques sont utilisés. Des zéolithes particulièrement adéquates sont les zéolithes de type A décrites dans le brevet US-A-2 882 243, qui, dans la plupart de leurs formes cationiques échangées notamment sous la forme calcium, présentent un diamètre de pore de l'ordre de 5Å et possèdent de fortes capacités pour adsorber les paraffines linéaires. Le terme diamètre de pore est conventionnel pour l'homme du métier. Il est utilisé pour définir de façon fonctionnelle la taille d'un pore en terme de taille de molécule capable d'entrer dans ce pore. Il ne désigne pas la dimension réelle du pore car celle-ci est souvent difficile à déterminer puisque souvent de forme irrégulière (c'est-à-dire non circulaire). D.W. Breck fournit une discussion sur le diamètre de pore effectif dans son livre intitulé "Zeolite Molecular Sieves" (John Wiley and Sons, New York, 1974) aux pages 633 à 641. D'autres tamis moléculaires incluant par exemple la zéolithe R (US-A-3 030 181), la zéolithe T (US-A-2 950 952), les silicoaluminophosphates et aluminophosphates (US-A4 440 871, 4 310 440 et 4 567 027), ainsi que les zéolithes naturelles, telles que la clinoptilolite, la chabazite et l'érionite conviennent pour effectuer les séparations mises en oeuvres dans l'unité 2 des versions 1 et 2. Enfin, l'utilisation de tamis comme la ferriérite (US-A-4 804 802 et 4 717 784), les zéolithes ZSM-5 (US-A-3 702 886), ZSM-11 (US-A-4 108 881), ZSM-23 (US-A-4 076 842) et ZSM-35 (US-A-4016245) et la silicalite (US-A-5 055 633) est aussi parfaitement adaptée aux séparations effectuées dans l'unité 2 des versions 1 et 2, puisque les propriétés diffusionnelles différentes des isomères en leur sein peuvent être exploitées. Les détails de l'adsorption des paraffines linéaires sur chacun de ces tamis est connu par l'homme de l'art et ne fera donc pas l'objet de plus de détail.

Dans le cadre de l'adsorption soit des paraffines monobranchées à partir du flux 5 riche en paraffines mono- et multibranchées, composés naphténiques et/ou aromatiques (unité 3, version 1), soit des paraffines monobranchées et linéaires à partir de la charge 1 (unité 3, version 2), on préférera pour l'application de l'invention des tamis moléculaires microporeux ayant un diamètre de pore effectif légèrement supérieur à 5 Å. Parmi eux se trouvent ceux possédant des pores de section elliptique de dimensions comprises entre 5,0 et 5,5 Å suivant le petit axe et environ 5,5 à 6,0 Å suivant le grand axe. Un adsorbant présentant ces caractéristiques et donc particulièrement adapté à la présente invention est la silicalite. Le terme silicalite inclut ici à la fois les silicopolymorphes décrits dans le brevet US-A-4 061 724 et aussi la silicalite F décrite dans le brevet US-A-4 073 865. D'autres adsorbants présentant ces mêmes caractéristiques et en conséquence particulièrement adaptés à la présente application sont la ZSM-5, la ZSM-11, ZSM-35 (US-A-4 016 245), ZSM-48 ainsi que de nombreuses autres aluminosilicates cristallins analogues. La ZSM-5 et la ZSM-11 sont décrites dans les brevets US-A-3 702 886, Re 29 948 et US-A-3 709 979. La teneur en silice de ces adsorbants peuvent être variables. Les adsorbants les plus adaptés à ce type de séparation sont ceux qui présentent des teneurs en silice élevées. Le rapport molaire Si/Al doit être de préférence au moins égal à 10 et de manière préférée supérieur à 100. Un autre type d'adsorbant particulièrement adapté à l'application de l'invention possède des pores de section elliptique de dimensions comprises entre 4,5 et 5,5 Å. Ce type d'adsorbant a été caractérisé par exemple dans le brevet US-A-4 717 748 comme étant un tectosilicate possédant des pores de taille intermédiaire entre celle des pores du tamis au calcium 5A et celle des pores de la ZSM-5. Les adsorbants préférés de cette famille incluent la ZSM-23 décrites dans le brevet US-A-4 076 872 et la ferriérite décrite dans les brevets US-A-4 016 425 et 4 251 499.

Ces différents adsorbants ont des tailles de pores telles que chacun des isomères des coupes C5-C8 ou des coupes intermédiaires peut être adsorbé. La cinétique de diffusion de ces isomères est cependant suffisamment différente pour être mise à profit. Dans certaines conditions de mise en oeuvre, ces tamis moléculaires permettront d'effectuer chacune des séparations correspondants aux unités 2 ou 3 des versions 1 et 2 de la présente invention.

Dans le cas où une des unités de séparation fonctionne à l'aide d'une technique de perméation, la membrane utilisée pourra prendre la forme de fibres creuses, faisceaux de tubes, ou d'un empilement de plaques. Ces configurations sont connues de l'homme de l'art, et permettent d'assurer la répartition homogène du fluide à séparer sur toute la surface de la membrane, d'entretenir une différence de pression de part et d'autre de la membrane, de recueillir séparément le fluide qui a perméé (le perméat) et celui qui n'a pas perméé (le rétentat). La couche sélective pourra être réalisée au moyen d'un des matériaux adsorbants précédemment décrits pourvu qu'il puisse constituer une surface uniforme délimitant une zone dans laquelle peut circuler au moins une partie de la charge, et une zone dans laquelle circule au moins une partie du fluide qui a perméé.

La couche sélective peut être déposée sur un support perméable assurant la résistance mécanique de la membrane ainsi constituée, comme décrit par exemple dans les demandes internationales WO-A-96/01 687 et 93/19 840.

Préférentiellement, la couche sélective est réalisée par croissance de cristaux de zéolithe à partir d'un support microporeux, tel que décrit dans les demandes de brevets EP-A-778 075 et 778 076

Selon un mode préféré de l'invention, la membrane est constitué par une couche continue de cristaux de silicalite d'environ 40 microns d'épaisseur, liée à un support en alumine alpha présentant une porosité de 200 nm.

Les conditions opératoires seront choisies de façon à maintenir sur toute la surface membranaire une différence de potentiel chimique du ou des constituants à séparer pour favoriser leur transfert à travers la membrane. Les pressions de part et d'autre de la membrane devront permettre de réaliser des écarts moyens de pressions partielles transmembranaires des constituants à séparer de 0,05 à 1,0 MPa.

Il est possible pour diminuer la pression partielle des constituants d'utiliser un gaz de balayage ou de maintenir le vide par une pompe à vide à une pression qui, selon les constituants, peut varier de 100 à 10⁴ Pa et de condenser les vapeurs à très basse température, typiquement vers - 40 °C. Selon les hydrocarbures utilisés, les températures ne devront pas excéder 200 à 400 °C, pour limiter les réactions de craquage et/ou de cokage des hydrocarbures oléfiniques et/ou aromatiques au contact de la membrane. De préférence, la vitesse de circulation de la charge doit être telle que son écoulement ait lieu en régime turbulent.

Les conditions opératoires des deux unités de séparation dépendent de leur mise en oeuvre, de l'adsorbant ou de la membrane considérés, ainsi que de la séparation à effectuer. Elles sont comprises entre 50°C et 450°C pour la température et de 0,01 à 7 MPa pour la pression. Plus précisément, si la séparation est effectuée en phase liquide, les conditions de séparation sont : 50°C à 200°C pour la température et 0,1 à 7 MPa pour la pression. Si ladite séparation est effectuée en phase gazeuse, ces conditions sont : 150°C à 450°C pour la température et 0,01 à 7 MPa pour la pression.

La version 1 du procédé conduit à minimiser, par rapport à la version 2 du procédé, la quantité d'adsorbant ou la surface de membrane nécessaire à la séparation dans l'unité 3. En effet, dans la version 2, la mise en oeuvre de l'unité 3 entraîne en fonction de la technique de séparation utilisée, soit l'adsorption de l'ensembles des paraffines linéaires et monobranchées, soit leur passage à travers la membrane. Dans la version 1, la mise en oeuvre de l'unité 3 entraîne la seule adsorption des composés monobranchés ou respectivement le passage à travers la membrane de ces seuls isomères (le perméat n'est constitué que de monobranchés).

Dans le cas où la charge du procédé comprend la coupe C5, de l'isopentane issu de cette coupe est retiré des flux traversant le procédé à l'aide d'un déisopentaniseur. Celui-ci peut être disposé soit sur la charge 1, soit sur les flux 5 ou 6 pour la version 1 ou sur les flux 1, 15 et 16 pour la version 2. Une telle mise en oeuvre permet d'optimiser la gestion des flux de ce procédé puisque l'isopentane ainsi séparé sert d'éluant ou de gaz de balayage respectivement pour les procédés de séparation par adsorption ou par perméation. La disposition du déisopentaniseur respectivement sur le flux 6 d'une part (version 1) et les flux 15 ou 16 d'autre part (version 2) montre bien que l'isopentane est préférentiellement séparé dans les conditions de fonctionnement des sections de séparation avec les composés monobranchés et non avec les composés multibranchés. L'invention incluant la déisopentanisation se distingue en conséquence nettement de celles reliées aux brevets US-A-5 055 633 et 5 055 634.

Il peut aussi être avantageux de disposer un dépentaniseur sur les flux 1 et/ou 4 pour la version 1 et sur les flux 1 et/ou 15 et/ou 14 pour la version 2. Si le dépentaniseur est situé sur les flux 1, pour la version 1 ou sur les flux 1 et 15 pour la version 2, il peut être suivi d'un déisopentaniseur dans le but de disposer dans le cadre du procédé soit d'un mélange isopentane/pentane soit de chacun de ces corps purs indépendamment. Dans ce cas, c'est l'isopentane seul ou en mélange avec du pentane qui sert d'éluant ou de gaz de balayage respectivement pour les procédés de séparation par adsorption ou par perméation.

Enfin, de la même façon, dans le cas de coupes ne contenant pas de C5 mais contenant des C6, le procédé comprend un déisohexaniseur placé sur les flux 1, 5 ou 6 pour la version 1 ou sur les flux 1, 15 et 16 pour la version 2. Une telle mise en oeuvre permet d'optimiser la gestion des flux de ce procédé puisque les composés monobranchés en C6 ainsi séparés servent d'éluant ou de gaz de balayage respectivement pour les procédés de séparation par adsorption ou par perméation.

### EXEMPLES:

### Exemple 1 : Procédé de séparation en trois effluents à l'aide de deux unités fonctionnant par adsorption en phase gazeuse

Pour illustrer l'invention dans sa version 1, on donne ci-après un exemple dans lequel les deux séparations sont effectuées par adsorption en phase gazeuse selon la technique du PSA, à partir d'une coupe de distillation directe C5-C8 comprenant des hydrocarbures paraffiniques, naphténiques, aromatiques et oléfiniques.

La charge fraîche du procédé possède la composition indiquée dans le. Tableau 2 et en conséquence un indice d'octane recherche de 73,1 et un indice d'octane moteur de 70,33.

**Tableau 2**

| Composants | masse % |
|---|---|
| ic4 | 0,01 |
| nC4 | 0,46 |
| nC5 | 9,10 |
| iC5 | 6,10 |
| cyclopentane | 0,61 |
| nC6 | 6,38 |
| monobranchés en C6 | 6,43 |
| dibranchés en C6 | 1,31 |
| cydohexane | 3,87 |
| méthylcyclopentane | 3,01 |
| nC7 | 6,23 |
| monobranchés en C7 | 4,18 |
| dibranchés en C7 | 2,43 |
| tribranchés en C7 | 0,46 |
| diméthylcycloC5 | 4,24 |
| méthylcycloC6 | 20,10 |
| nC8 | 2,91 |
| monobranchés C8 | 2,18 |
| dibranchés C8 | 1,31 |
| tribranchés C8 | 0,64 |
| triméthylcycloC5 | 6,00 |
| éthylbenzene | 0,92 |
| toluene | 10,00 |
| Benzène | 1,16 |

La charge fraîche arrive par la ligne 1 avec un débit de 26,29 kg/h. Cette charge est déisopentanisée dans un premier déisopentaniseur. La fraction légère recueillie en tête de ce premier déisopentaniseur n°1 a la composition indiquée dans le Tableau 3, un indice d'octane recherche de 92,4 et un débit de 1,73 kg/h. Cette fraction est utilisée par la suite comme gaz de balayage du procédé PSA de l'unité de séparation 2.

**Tableau 3**

| Composant | masse % |
|---|---|
| nC4 | 7 |
| iC4 | 0,15 |
| iC5 | 92,85 |

La charge déisopentanisée, préalablement réchauffée à 250°C et à une pression de 1,4 MPa, arrive dans l'unité de séparation 2. Cette unité comprend 4 adsorbeurs qui sont des cylindres de 0,053 m de diamètre interne et de 4,77 m de hauteur, contenant chacun 8,05 kg de tamis moléculaire 5A (ou zéolithe 5A), mis sous forme de billes de 1,2 mm de diamètre. La charge et le désorbant alimentent l'unité de séparation sous contrôle de débit et les effluents sont recueillis sous contrôle de pression. Dans le PSA à quatre adsorbeurs, chacun des lits d'adsorption subit cycliquement les étapes suivantes :
1 - Pressurisation : la charge déisopentanisée (24,66 kg/h) pénètre dans le lit qui contient du gaz de désorption à basse pression. Au fur et à mesure de cette introduction de charge, la pression monte dans l'adsorbeur jusqu'à atteindre la pression d'adsorption 1,4 MPa.
2- Adsorption : la charge est envoyée à co-courant de l'étape de pressurisation dans le lit et les paraffines linéaires sont sélectivement adsorbées sur la zéolithe 5A, alors que les paraffines mono- et multibranchées et les composés aromatiques et naphténiques sont produits comme effluent de cet adsorbeur à haute pression.
3 - Dépressurisation : lorsque l'adsorbant est suffisamment chargé de paraffines linéaires, une étape de dépressurisation à 0,3 MPa est mise en oeuvre à co-courant des étapes de pressurisation et d'adsorption. Lors de cette étape, une grande partie paraffines mono- et multibranchées contenues dans les volumes morts de l'adsorbeur sont produits.
4 - Stripping par gaz de balayage : la fraction légère produite par les déisopentaniseurs n°1 et 2, est utilisée comme gaz de balayage pour désorber la majorité des paraffines linéaires du tamis 5A.

Le fonctionnement décrit ci-dessus est celui d'un des adsorbeurs. Les quatre adsorbeurs qui forment l'unité de séparation 2 fonctionnent de la même manière mais de façon décalée afin de conduire à la production continue de deux effluents. Le flux de stripping contenant les paraffines linéaires et l'isopentane est produit avec un débit de 18,95 kg/h et un indice d'octane recherche de 69,47. Ce flux est alors envoyé vers le déisopentanseur n°2 afin d'obtenir, d'une part, de l'isopentane, qui est recyclé comme gaz de balayage vers l'unité de séparation 2, avec un débit de 12,3 kg/h et, d'autre part, les paraffines linéaires recherchées (flux 4) avec un débit de 6,65 kg/h et un indice d'octane recherche de 27 (composition donnée dans le Tableau 4). Ce flux 4 contient des traces de paraffines monobranchées à une teneur de 5 %. Le flux de production 5 riche en paraffines mono- et multibranchées, et en composés naphténiques et/ou aromatiques, est produit avec un débit de 16,64 kg/h et un indice d'octane de 89,9. Ce flux contient 9 % d'isopentane et 0,25 % de paraffines linéaires. Le flux 5 est alors envoyé vers l'unité de séparation 3. Cette unité fonctionne aussi suivant la technique de séparation PSA. Elle comprend 4 adsorbeurs qui sont des cylindres de 0,04 m de diamètre interne et de 5,7 m de hauteur, contenant chacun 5,47 kg de silicalite, mise sous forme de billes de 1,2 mm de diamètre. Chacun des lits d'adsorption de cette unité 3 subit cycliquement les mêmes étapes que celles décrites dans le cadre de la séparation effectuées dans l'unité 2. Pendant les étapes de pressurisation et d'adsorption, les adsorbeurs sont alimentés par un flux riche en paraffines mono-, multibranchées, composés naphténiques et/ou aromatiques (flux 5). Dans les conditions de mise en oeuvre de la séparation, les paraffines monobranchées vont s'adsorber préférentiellement sur la silicalite en déplaçant l'isopentane adsorbé présent dans l'adsorbeur suite à l'étape de stripping. Dans les conditions de fonctionnement de l'adsorbeur, les paraffines multibranchées, les composés aromatiques et naphténiques ne sont pas adsorbés et sont produits comme effluent de l'adsorbeur à haute pression (1,4 MPa). L'étape de dépressurisation à 0,3 MPa à co-courant permet de produire une grande partie des composés non adsorbés contenus dans les volumes morts de l'adsorbeur. Enfin, une fraction de l'isopentane issu du déisopentaniseur n°3, est alors utilisé comme gaz de balayage pour désorber la majorité des paraffines monobranchées de la silicalite. L'isopentane permet d'une part d'abaisser la pression partielle des composés monobranchés adsorbés et permet de plus de déplacer ces composés, en raison de sa propre adsorption par la silicalite. Les quatre adsorbeurs qui forment l'unité de séparation 3 fonctionnent de la même manière mais de façon décalée afin de conduire à la production continue de deux effluents. Le flux de balayage contenant les paraffines monobranchées et l'isopentane est produit avec un débit de 10,58 kg/h. Il contient 5,48 % de paraffines dibranchées et présente un indice d'octane de 82,9. Ce flux est alors envoyé vers le déisopentaniseur n°3 afin d'obtenir, d'une part, de l'isopentane, qui est recyclé comme gaz de désorption vers l'unité de séparation 3 et, d'autre part, les paraffines monobranchées recherchées (flux 6 : débit 3,6 kg/h, indice d'octane 65,3, composition donnée dans le Tableau 4). Le flux de production 7, riche en paraffines multibranchées et en composés naphténiques et/ou aromatiques est produit avec un débit de 15,17 kg/h. Ce flux comporte de plus 5,9% d'isopentane, 0,4 % de paraffines monobranchées. Sa composition est donnée dans le Tableau 4 et son indice d'octane est de 92,8.

Ce procédé requiert le recyclage en boucle fermée d'une certaine quantité d'isopentane entre les déisopentaniseurs n°2 et n°3 et les unités de séparation 2 et 3. Le débit de ce gaz de désorption peut être ajusté en fonction des spécifications des unités de séparation. Une partie de ce gaz de désorption circulant en boucle fermée peut être récupérée
- dans les flux 4, 6, 7 et préférentiellement dans le flux 7 contenant les paraffines dibranchés les composés naphténiques et/ou aromatiques.
- dans les fractions légères des déisopentaniseurs n°2 et n°3.

Cette quantité de gaz de désorption ainsi prélevée correspond à la quantité de la fraction légère prélevée par le déisopentaniseur n°1 de la charge fraîche dont la composition est donnée dans le Tableau 3. Dans le cas de l'exemple 1, 52 % de l'isopentane introduit dans la charge est dans le flux 7, l'essentiel de la quantité restante est prélevée en tête du déisopentaniseur n°3.

**Tableau 4**

| Composition du flux 4 riche en paraffines linéaires | (% masse) | Composition du flux 6 riche en paraffines monobranchées | (% masse) | Composition du flux 7 riche en paraffines multibranchées | (% masse) |
|---|---|---|---|---|---|
| nC5 | 33,8 | | | isopentane | 5,9 |
| nC6 | 25,2 | monobranchés en C6 | 42,5 | dibranchés en C6 | 2,27 |
| nC7 | 24,6 | monobranchés en C7 | 27,52 | dibranchés en C7 | 4,22 |
| nC8 | 11 ,4 | monobranchés en C8 | 14,2 | dibranchés en C8 | 2,27 |
| composés monobranchés | 5 | composés dibranchés | 15,7 | composés monobranchés | 0,45 |
| | | linéaires | 0,08 | naphtènes | 64,2 |
| | | | | aromatiques | 20,7 |
| **RON** | **27** | | **65,3** | | **92,8** |

Globalement, le procédé suivant l'invention conduit à la production de trois effluents respectivement riches en paraffines linéaires, en paraffines monobranchées et en paraffines multibranchées, composés naphténiques et/ou aromatiques, à partir d'une coupe de distillation directe C5-C8 comprenant des hydrocarbures paraffiniques, naphténiques et/ou aromatiques.

### Exemple 2 : Procédé de séparation en trois effluents à l'aide de deux réacteurs, l'un fonctionnant par adsorption en phase liquide et l'autre par perméation gazeuse

Pour illustrer l'invention dans sa version 2, on donne ci-après un exemple dans lequel la première étape de séparation est effectuée par adsorption en phase liquide selon la technique du contre-courant simulé, à partir d'une coupe de distillation directe C5-C8 comprenant des hydrocarbures paraffiniques, naphténiques, aromatiques et oléfiniques.

La charge fraîche du procédé possède la composition indiquée dans le Tableau 5 et en conséquence un indice d'octane recherche de 65,06 et un indice d'octane moteur de 63,53.

**Tableau 5**

| Composants | masse % |
|---|---|
| ic4 | 0,02 |
| nC4 | 0,91 |
| nC5 | 15,23 |
| iC5 | 9,50 |
| cyclopentane | 0,73 |
| nC6 | 15,80 |
| monobranchés en C6 | 12,61 |
| dibranchés en C6 | 5,30 |
| cyclohexane | 2,34 |
| méthylcyclopentane | 3,27 |
| nC7 | 7,45 |
| monobranchés en C7 | 3,95 |
| dibranchés en C7 | 1,06 |
| tribranchés en C7 | 1,20 |
| diméthylcycloC5 | 4,58 |
| méthylcycloC6 | 3,79 |
| nC8 | 1,12 |
| monobranchés C8 | 0,93 |
| di branchés C8 | 0,77 |
| tribranchés C8 | 0,28 |
| triméthylcycloC5 | 4,03 |
| éthylbenzene | 0,99 |
| toluene | 3,73 |
| Benzène | 0,41 |

La charge fraîche arrive par la ligne 1 avec un débit de 25,75 kg/h. Cette charge est déisopentanisée dans un premier déisopentaniseur. La fraction légère recueillie en tête de ce premier déisopentaniseur (n°1) a la composition indiquée dans le Tableau 6, un indice d'octane recherche de 92,4 et un débit de 2,68 kg/h. Cette fraction est utilisée par la suite comme gaz de balayage dans l'unité de perméation gazeuse (unité de séparation 2).

**Tableau 6**

| Composant | masse % |
|---|---|
| nC4 | 8,75 |
| iC4 | 0,15 |
| iC5 | 91,1 |

La charge déisopentanisée, préalablement réchauffée à 100°C et à une pression de 1,8 MPa, alimente l'unité de séparation 3, qui consiste en une unité d'adsorption fonctionnant en contre-courant simulé (CCS). Cette unité comprend plusieurs colonnes en série constituées de cylindres de 0,1 m de diamètre interne. L'unité complète à une longueur de 15 m et contient 95 kg de silicalite, mise sous forme de billes de 0,7 mm de diamètre. La charge et le désorbant (provenant des déisopentaniseurs n°2 et n°3 qui seront décrits plus loin) alimentent l'unité de séparation 3 fonctionnant sous contrôle de débit (respectivement 23,07 kg/h et 57,65 kg/h) et les effluents sont recueillis sous contrôle de pression. Dans l'unité CCS, la charge déisopentanisée (23,07 kg/h) pénètre dans le lit. Les paraffines linéaires et monobranchées sont alors adsorbées par la silicalite en déplaçant l'isopentane adsorbé. Dans les conditions de mise en oeuvre, les paraffines multibranchées, les composés aromatiques et naphténiques ne sont pas adsorbés. Les points d'injection de la charge, du raffinat et de l'extrait sont continûment déplacés. Ce procédé permet de produire un flux riche en paraffines dibranchées, composés naphténiques et/ou aromatiques et isopentane avec un débit de 29,26 kg/h et un indice d'octane de 94,16. Ce flux est déisopentanisé dans le déisopentaniseur n°2 de façon à recycler l'isopentane vers l'unité de séparation 3. L'isopentane recueilli en phase liquide dans le condenseur de tête de la colonne du déisopentaniseur n°2 est envoyé d'une part, en reflux de la colonne et, d'autre part, au recyclage de l'éluant de l'unité de séparation 3. Ce recyclage a un débit de 20,9 kg/h. En fond de ce deuxième déisopentaniseur, on recueille un flux 17 riche en paraffines dibranchées, en naphténiques et/ou en aromatiques dont la composition simplifiée est donnée dans le Tableau 7.

Le flux issu de l'unité de séparation 3 contenant les paraffines linéaires, monobranchées et une partie du désorbant est produit avec un débit de 51,45 kg/h et un indice d'octane recherche de 78,47. Il contient 71 % en masse d'isopentane.

Ce flux est alors envoyé vers un troisième déisopentaniseur afin d'obtenir :
● d'une part, en tête de colonne, de l'isopentane, qui est en partie recyclé comme éluant vers l'unité de séparation 3, avec un débit de 36,75 kg/h et
● d'autre part, en fond de colonne, les paraffines linéaires et monobranchées recherchées (flux 15) avec un débit de 14,7 kg/h et un indice d'octane recherche de 42,9. Ce flux contient des traces de paraffines multibranchées à une teneur de 5 % en masse.

Ce flux est détendu à une pression de 0,2 MPa et 100°C afin d'alimenter l'unité de séparation 2 consistant en une unité de perméation gazeuse. Cette unité est constituée d'un faisceau de tubes en alumine dont la surface interne est recouverte d'une couche de silicalite de 20 microns d'épaisseur. La surface totale utile de la membrane est 5 m². La charge gazeuse est distribuée à l'intérieur des tubes, le gaz de balayage provenant du premier déisopentaniseur et du quatrième déisopentaniseur (décrit plus loin) est introduit après détente à pression atmosphérique et réchauffage à 100 °C dans la calandre du perméateur et est recueilli à une autre extrémité avec les paraffines linéaires. L'introduction et le soutirage du gaz de balayage de part et d'autre de la calandre du perméateur sont réalisés de telle sorte que les fluides de charge et de perméat circulent à contre-courant. Les vitesses de circulation desdits fluides sont choisies de façon à maintenir leur écoulement en régime turbulent.

Les paraffines linéaires s'adsorbent préférentiellement dans les cavités de la zéolithe (silicalite), et diffusent sous l'effet de leur gradient de potentiel chimique maintenu de part et d'autre de la membrane par les conditions opératoires précitées. La charge appauvrie en paraffines linéaires récupérée en sortie du perméateur (flux 16: débit de 4,39 kg/h) contient 7,8 % en masse de paraffines linéaires et 6,8 % en masse d'isopentane. La composition du flux 16 est reportée dans le Tableau 7. Le gaz de balayage, au cours de sa circulation dans le perméateur, se charge en paraffines linéaires et en une faible quantité de paraffines monobranchées ayant aussi perméé à travers la membrane. Il sort du perméateur avec un débit de 15,54 kg/h, avec une proportion de 31,2 % en masse d'isopentane Ce flux est envoyé au déisopentaniseur n°4, où l'isopentane est prélevé en tête. Une partie de cet isopentane est envoyée au reflux du déisopentaniseur n°4; une autre partie (2,3 kg/h) sous forme vapeur est réchauffée et, réunie avec le flux de tête du déisopentaniseur n°1, introduite comme gaz de balayage dans le perméateur, sous un débit de 5 kg/h. Par ailleurs, une purge en tête de ce déisopentaniseur permet d'extraire un flux de 2,4 kg/h. Le fond du déisopentaniseur n°4 produit, sous un débit de 10,61 kg/h, un flux 14 riche en paraffines linéaires, dont la composition est donnée dans le Tableau 7 ci-après.

**Tableau 7**

| Composition du flux 14 riche en paraffines linéaires | (% masse) | Composition du flux 16 riche en paraffines monobranchées | (% masse) | Composition du flux 17 riche en paraffines dibranchées | (% masse) |
|---|---|---|---|---|---|
| nC5 | 35,93 | isopentane | 6,8 | | |
| nC6 | 37,27 | monobranchés en C6 | 60,3 | dibranchés en C6 | 16,1 |
| nC7 | 17,57 | monobranchés en C7 | 19,3 | di et tri branchés en C7 | 6,4 |
| nC8 | 2,65 | monobranchés en C8 | 4,5 | di et tri branchés en C8 | 3,2 |
| composés monobranchés | 6,57 | composés dibranchés | 1,5 | composés monobranchés | 0,5 |
| | | composés linéaires | 7,6 | naphtènes | 58 |
| | | | | aromatiques | 15,8 |
| **RON** | **35** | **RON** | **65,3** | **RON** | **94** |

La quantité du recyclage d'isopentane qui circule en boucle fermée entre le perméateur et le déisopentaniseur est une variable du procédé. Pour une même surface de membrane installée, il est possible de fonctionner avec des rapports de débit de gaz de balayage sur la charge compris entre 0 et 3. Lorsque ce rapport augmente, la quantité de paraffines linéaires perméant à travers la membrane augmente, l'augmentation ayant lieu au détriment de la pureté des paraffines linéaires extraites.

Globalement le procédé suivant l'invention conduit à la production de trois effluents respectivement riches en paraffines linéaires, en paraffines monobranchées et en paraffines multibranchées, composés naphténiques et/ou aromatiques, à partir d'une coupe de distillation directe C5-C8 comprenant des hydrocarbures paraffiniques, naphténiques et/ou aromatiques.

### Exemple 3 : Procédé de séparation en trois effluents à l'aide de deux unités fonctionnant par adsorption en phase gazeuse

Pour illustrer l'invention dans sa version 2, on donne ci-après un exemple dans lequel les deux séparations sont effectuées par adsorption en phase gazeuse selon la technique du PSA, à partir d'une coupe de distillation directe C5-C8 comprenant des hydrocarbures paraffiniques, naphténiques, aromatiques et oléfiniques.

La charge fraîche du procédé possède la composition indiquée dans le Tableau 8 et en conséquence un indice d'octane recherche de 73,1 et un indice d'octane moteur de 70,33.

**Tableau 8**

| Composants | masse % |
|---|---|
| ic4 | 0,01 |
| nC4 | 0,46 |
| nC5 | 9,10 |
| iC5 | 6,10 |
| cyclopentane | 0,61 |
| nC6 | 6,38 |
| monobranchés en C6 | 6,43 |
| dibranchés en C6 | 1,31 |
| cyclohexane | 3,87 |
| méthylcydopentane | 3,01 |
| nC7 | 6,23 |
| monobranchés en C7 | 4,18 |
| dibranchés en C7 | 2,43 |
| tribranchés en C7 | 0,46 |
| diméthylcydoC5 | 4,24 |
| méthylcycloC6 | 20,10 |
| nC8 | 2,91 |
| monobranchés C8 | 2,18 |
| dibranchés C8 | 1,31 |
| tribranchés C8 | 0,64 |
| triméthylcycloC5 | 6,00 |
| éthylbenzene | 0,92 |
| toluene | 10,00 |
| Benzène | 1,16 |

La charge fraîche arrive par la ligne 1 avec un débit de 26,29 kg/h. Cette charge est déisopentanisée dans un premier déisopentaniseur. La fraction légère recueillie en tête de ce premier déisopentaniseur n°1 a la composition indiquée dans le Tableau 9, un indice d'octane recherche de 92,4 et un débit de 2,44 kg/h. Cette fraction est utilisée par la suite comme gaz de balayage du procédé PSA de l'unité de séparation 2.

**Tableau 9**

| Composant | masse % |
|---|---|
| nC4 | 4,8 |
| iC4 | 0,10 |
| iC5 | 95,1 |

La charge déisopentanisée, préalablement réchauffée à 250°C et à une pression de 1,4 MPa, arrive dans l'unité de séparation 2. Cette unité comprend 4 adsorbeurs qui sont des cylindres de 0,3 m de diamètre interne et de 2,2 m de hauteur, contenant chacun 108 kg de silicalite, mise sous forme de billes de 1,2 mm de diamètre. La charge et le désorbant alimentent l'unité de séparation sous contrôle de débit et les effluents sont recueillis sous contrôle de pression. Dans le PSA à quatre adsorbeurs, chacun des lits d'adsorption subit cycliquement les étapes suivantes :
1 - Pressurisation : la charge déisopentanisée (23,86 kg/h) pénètre dans le lit qui contient du gaz de désorption à basse pression. Au fur et à mesure de cette introduction de charge, la pression monte dans l'adsorbeur jusqu'à atteindre la pression d'adsorption 1,4 MPa.
2 - Adsorption : la charge est envoyée à co-courant de l'étape de pressurisation dans le lit et les paraffines linéaires et monobranchées sont sélectivement adsorbées sur la silicalite alors que les paraffines multibranchées et les composés aromatiques et naphténiques sont produits comme effluent de cet adsorbeur à haute pression.
3 - Dépressurisation : lorsque l'adsorbant est suffisamment chargé de paraffines linéaires et monobranchées, une étape de dépressurisation à 0,3 MPa est mise en oeuvre à co-courant des étapes de pressurisation et d'adsorption. Lors de cette étape, une grande partie paraffines multibranchées contenues dans les volumes morts de l'adsorbeur sont produits.
4 - Stripping par gaz de balayage : la fraction légère produite par les déisopentaniseurs n°1 et 2, est utilisée comme gaz de balayage pour désorber la majorité des paraffines linéaires et monobranchées.

Le fonctionnement décrit ci-dessus est celui d'un des adsorbeurs. Les quatre adsorbeurs qui forment l'unité de séparation 2 fonctionnent de la même manière mais de façon décalée afin de conduire à la production continue de deux effluents. Le flux de production 5 riche en paraffines multibranchées et en composés naphténiques et/ou aromatiques, est produit avec un débit de 14,2 kg/h et un indice d'octane de 86,1. Ce flux contient 4,4 % d'isopentane et 6,3 % de paraffines linéaires et monobranchées. Le flux de stripping contenant les paraffines linéaires et monobranchées et l'isopentane est produit avec un débit de 11,8 kg/h. Ce flux est alors envoyé vers le déisopentaniseur n°2 afin d'obtenir, d'une part, de l'isopentane, qui est recyclé comme gaz de balayage vers l'unité de séparation 2, avec un débit de 1,5 kg/h et, d'autre part, les paraffines linéaires et monobranchées recherchées (flux 4) avec un débit de 10,3 kg/h et un indice d'octane recherche de 42,75 (composition donnée dans le Tableau 10). Ce flux 4 contient des traces de paraffines multibranchées, composés aromatiques et naphténiques à une teneur de 14 %.

Le flux 4 est alors envoyé vers l'unité de séparation 3. Cette unité fonctionne aussi suivant la technique de séparation PSA. Elle comprend 4 adsorbeurs qui sont des cylindres de 0,25 m de diamètre interne et de 2 m de hauteur, contenant chacun 70 kg de tamis moléculaire 5A (zéolithe 5A), mis sous forme de billes de 1,2 mm de diamètre. Chacun des lits d'adsorption de cette unité 3 subit cycliquement les mêmes étapes que celles décrites dans le cadre de la séparation effectuées dans l'unité 2. Pendant les étapes de pressurisation et d'adsorption, les adsorbeurs sont alimentés par un flux riche en paraffines linéaires et monobranchées (flux 4). Les paraffines linéaires vont s'adsorber préférentiellement sur la zéolithe 5A en déplaçant l'isopentane adsorbé présent dans l'adsorbeur suite à l'étape de stripping. Dans les conditions de fonctionnement de l'adsorbeur, les paraffines monobranchées ne sont pas adsorbées et sont produites comme effluent de l'adsorbeur à haute pression (1,4MPa). L'étape de dépressurisation à 0,3 MPa à co-courant permet de produire une grande partie des composés non adsorbés contenus dans les volumes morts de l'adsorbeur. Enfin, une fraction de l'isopentane issu des déisopentaniseurs n°1, n°2 ou n°3, est alors utilisé comme gaz de balayage pour désorber la majorité des paraffines linéaires de la zéolithe 5A. Les quatre adsorbeurs qui forment l'unité de séparation 3 fonctionnent de la même manière mais de façon décalée afin de conduire à la production continue de deux effluents. Le flux de balayage contenant les paraffines linéaires et l'isopentane est produit avec un débit de 5,75kg/h. Il contient 13,66% de paraffines multibranchées, composés aromatiques et naphténiques. Ce flux est alors envoyé vers le déisopentaniseur n°3 afin d'obtenir, d'une part, de l'isopentane, qui est recyclé comme gaz de désorption vers les unités de séparation 2 et 3 et, d'autre part, les paraffines linéaires recherchées (flux 6 : débit 5,2 kg/h, indice d'octane 22.4, composition donnée dans le Tableau 10). Le flux de production 7, riche en paraffines monobranchées est produit avec un débit de 7kg/h. Ce flux comporte de plus 26.85 % d'isopentane. Sa composition est donnée dans le Tableau 10 et son indice d'octane est de 72,6.

Ce procédé requiert le recyclage en boucle fermée d'une certaine quantité d'isopentane entre les déisopentaniseurs n° 1, n° 2 et n° 3 et les unités de séparation 2 et 3. Le débit de ce gaz de désorption peut être ajusté en fonction des spécifications des unités de séparation. Une partie de ce gaz de désorption circulant en boucle fermée peut être récupérée
- dans les flux 5, 6 ou 7.
- dans les fractions légères des déisopentaniseurs n°2 et n°3.

Cette quantité de gaz de désorption ainsi prélevée correspond à la quantité de la fraction légère prélevée par le déisopentaniseur n°1 de la charge fraîche dont la composition est donnée dans le Tableau 9.

**Tableau 10**

| Composition du flux 6 riche en paraffines linéaires | (% masse) | Composition du flux 7 riche en paraffines monobranchées | (% masse) | Composition du flux 5 riche en paraffines multibranchées, aromatiques et naphténiques | (% masse) |
|---|---|---|---|---|---|
| nC5 | 27 ,1 | iC5 | 26,8 | isopentane | 4,4 |
| nC6 | 28,32 | monobranchés en C6 | 22,6 | multibranchés | 10,3 |
| nC7 | 27.65 | monobranchés en C7 | 14,7 | aromatiques | 20,2 |
| nC8 | 12.9 | monobranchés en C8 | 7,65 | naphtènes | 63,3 |
| mono- et multibranchés | 4 | linéaires et multibranchés | 28 | linéaires et monobranchés | 1,9 |
| **RON** | **22** | | **72,6** | | **86,1** |

Globalement, le procédé suivant l'invention conduit à la production de trois effluents respectivement riches en paraffines linéaires, en paraffines monobranchées et en paraffines multibranchées, composés naphténiques et/ou aromatiques, à partir d'une coupe de distillation directe C5-C8 comprenant des hydrocarbures paraffiniques, naphténiques et/ou aromatiques.

## Revendications

1. Procédé de séparation de coupes légères C5-C8, C5-C7, C6-C8 ou C6-C7, comprenant des hydrocarbures paraffiniques, et éventuellement naphténiques, aromatiques et/ou oléfiniques, comprenant la mise en oeuvre d'au moins deux unités de séparation disposées en série, de manière à obtenir trois effluents, respectivement un effluent riche en paraffines linéaires, un effluent riche en paraffines monobranchées et un effluent riche en paraffines dibranchées, tribranchées et éventuellement en composés naphténiques et/ou aromatiques, chaque unité de séparation fonctionnant par adsorption ou par perméation, ledit procédé étant **caractérisé en ce que** de l'isopentane ou de l'isohexane contenu dans ladite charge est séparé de celle-ci et sert d'éluant pour la régénération de la (ou des) unités de séparation par adsorption et/ou de gaz de balayage pour la régénération de la (ou des) unités de séparation par perméation.

2. Procédé selon la revendication 1, **caractérisé en ce que** l'on envoie la charge dans une première unité de séparation, où sont séparées les paraffines linéaires des paraffines ramifiées, ladite unité produisant une charge dénormalisée, qui est envoyée vers la seconde unité de séparation, où sont séparés d'une part les paraffines monobranchées et d'autre part les paraffines multibranchées et éventuellement les composés naphténiques et/ou aromatiques.

3. Procédé selon la revendication 1, **caractérisé en ce que** l'on envoie la charge dans une première unité de séparation, où sont séparés d'une part les paraffines multibranchées et éventuellement les composés naphténiques et/ou aromatiques et d'autre part les paraffines linéaires et monobranchées, qui sont envoyées vers la seconde unité de séparation où sont séparés deux effluents, l'un riche en paraffines monobranchées, l'autre riche en paraffines linéaires.

4. Procédé selon l'une des revendications 1 à 3, **caractérisé en ce que** la charge comprend la coupe C5 et l'isopentane issu de cette coupe est séparé avec les paraffines monobranchées,

5. Procédé selon l'une des revendications 1 à 3, **caractérisé en ce que** la charge comprend la coupe C5 et l'isopentane issu de cette coupe est retiré des flux traversant le procédé à l'aide d'un déisopentaniseur disposé en amont de l'une (ou des) unité(s) de séparation.

6. Procédé selon l'une des revendications 1 à 3, **caractérisé en ce que** la charge comprend la coupe C5 et l'isopentane issu de cette coupe est retiré des flux traversant le procédé à l'aide d'un déisopentaniseur disposé en aval des unités de séparation.

7. Procédé selon l'une des revendications 1 à 6, **caractérisé en ce qu'**il met en oeuvre au moins deux unités de séparation fonctionnant par adsorption.

8. Procédé selon l'une des revendications 1 à 6, **caractérisé en ce qu'**il met en oeuvre au moins une unité de séparation fonctionnant par adsorption et au moins une unité de séparation par perméation utilisant une ou plusieurs membranes.

9. Procédé selon l'une des revendications 1 à 6, **caractérisé en ce qu'**il met en au moins deux unités de séparation par perméation utilisant une ou plusieurs membranes.

10. Procédé selon l'une des revendications 1 à 9, **caractérisé en ce que** la charge est issue de la distillation atmosphérique.

11. Procédé selon l'une des revendications 1 à 9, **caractérisé en ce que** la charge est issue d'une unité de réformage.

12. Procédé selon la revendication 11, **caractérisé en ce que** la charge est un réformat léger.

13. Procédé selon l'une des revendications 1 à 9, **caractérisé en ce que** la charge est issue d'une unité de conversion.

14. Procédé selon la revendication 13, **caractérisé en ce que** la charge est un naphta d'hydrocraquage.

## Patentansprüche

1. Verfahren zur Trennung leichter C5-C8-, C5-C7-, C6-C8- oder C6-C7-Fraktionen, die paraffinische und gegebenenfalls naphtenische, aromatische und/oder olefinische Kohlenwasserstoffe umfassen, das den Einsatz wenigstens zweier in Reihe angeordneter Trenneinheiten derart umfasst, dass drei Abströme, jeweils ein an linearen Paraffinen reicher Abstrom, ein an monoverzweigten Paraffinen reicher Abstrom und ein an diverzweigten, triverzweigten Paraffinen und gegebenenfalls an naphtenischen und/oder aromatischen Verbindungen reicher Abstrom, erhalten werden, wobei jede Trenneinheit durch Adsorption oder Permeation arbeitet, wobei das Verfahren **dadurch gekennzeichnet** t ist, dass in der Beschickung enthaltenes Isopentan oder Isohexan von jener getrennt wird und als Elutionsmittel für die Regenerierung der Trenneinheit (oder Trenneinheiten) durch Adsorption und/oder als Spülgas zur Regenerierung der Trenneinheit (oder Trenneinheiten) durch Permeation dient.

2. Verfahren nach Anspruch 1, **dadurch gekennzeichnet, dass** man die Beschickung in die erste Trenneinheit schickt, wo die linearen von den verzweigten Paraffinen getrennt werden, wobei die Einheit eine abnormalisierte Beschickung erzeugt, die zur zweiten Trenneinheit geschickt wird, wo einerseits die monoverzweigten Paraffine und andererseits die mehrfachverzweigten Paraffine und gegebenenfalls naphtenischen und/oder aromatischen Verbindungen getrennt werden.

3. Verfahren nach Anspruch 1, **dadurch gekennzeichnet, dass** man die Beschickung in die erste Trenneinheit schickt, wo einerseits die mehrfachverzweigten Paraffine und gegebenenfalls naphtenischen und/oder aromatischen Verbindungen und andererseits die linearen und monoverzweigten Paraffine getrennt werden, die zur zweiten Trenneinheit geschickt werden, wo zwei Abströme, der eine reich an monoverzweigten Paraffinen, der andere reich an linearen Paraffinen getrennt werden.

4. Verfahren nach einem der Ansprüche 1 bis 3, **dadurch gekennzeichnet, dass** die Beschickung die C5-Fraktion umfasst und dass isopentan aus dieser Fraktion mit den monoverzweigten Paraffinen getrennt wird.

5. Verfahren nach einem der Ansprüche 1 bis 3, **dadurch gekennzeichnet, dass** die Beschickung die C5-Fraktion umfasst und dass Isopentan aus dieser Fraktion aus den das Verfahren durchlaufenden Strömen mit Hilfe eines Deisopentanisators abgezogen wird, der vor der einen (oder den) Trenneinheit (oder Trenneinheiten) angeordnet ist.

6. Verfahren nach einem der Ansprüche 1 bis 3, **dadurch gekennzeichnet, dass** die Beschickung die C5-Fraktion umfasst und Isopentan aus dieser Fraktion aus den das Verfahren durchlaufenden Strömen mit Hilfe eines Deisopentanisators abgezogen wird, der nach den Trenneinheiten angeordnet ist.

7. Verfahren nach einem der Ansprüche 1 bis 6, **dadurch gekennzeichnet, dass** es wenigstens zwei durch Adsorption arbeitende Trenneinheiten einsetzt.

8. Verfahren nach einem der Ansprüche 1 bis 6, **dadurch gekennzeichnet, dass** es wenigstens eine durch Adsorption arbeitende Trenneinheit und wenigstens eine durch Permeation arbeitende Trenneinheit einsetzt, die eine oder mehrere Membranen verwendet.

9. Verfahren nach einem der Ansprüche 1 bis 6, **dadurch gekennzeichnet, dass** es wenigstens zwei Trenneinheiten durch Permeation einsetzt, die eine oder mehrere Membranen verwenden.

10. Verfahren nach einem der Ansprüche 1 bis 9, **dadurch gekennzeich-net, dass** die Beschickung aus der atmosphärischen Destillation stammt.

11. Verfahren nach einem der Ansprüche 1 bis 9, dadurch gekennzeichn e t, dass die Beschickung aus einer Reformierungseinheit stammt.

12. Verfahren nach Anspruch 11, **dadurch gekennzeichnet, dass** die Beschickung ein leichtes Reformat ist.

13. Verfahren nach einem der Ansprüche 1 bis 9, **dadurch gekennzeichnet, dass** die Beschickung aus einer Umwandlungseinheit stammt.

14. Verfahren nach Anspruch 13, **dadurch gekennzeichnet, dass** die Beschickung ein Hydrocracknaphta ist.

## Claims

1. A process for separating light C5-C8, C5-C7, C6-C8 or C6-C7 cuts, comprising paraffinic hydrocarbons, and possibly naphthenic, aromatic and/or olefinic hydrocarbons, comprising using at least two separation units disposed in series, so as to obtain three effluents, respectively an effluent which is rich in straight chain paraffins, an effluent which is rich in mono-branched paraffins and an effluent which is rich in di-branched paraffins, tri-branched paraffins and possibly in naphthenic and/or aromatic compounds, each separation unit working by adsorption or by permeation, said process being **characterized in that** isopentane or isohexane contained in said feed is separated therefrom and acts as an eluant to regenerate the adsorption separation unit or units and/or as a flushing gas to regenerate the permeation separation unit or units.

2. A process according to claim 1, **characterized in that** the feed is sent to a first separation unit where the straight chain paraffins are separated from the branched paraffins, said unit producing a denormalised feed which is sent to the second separation unit where the mono-branched paraffins are separated from the multi-branched paraffins and possibly the naphthenic and/or aromatic compounds.

3. A process according to claim 1, **characterized in that** the feed is sent to a first separation unit where the multi-branched paraffins and possibly the naphthenic and/or aromatic compounds are separated from the straight chain and mono-branched paraffins which are sent to the second separation unit where two effluents are separated, one which is rich in mono-branched paraffins, the other which is rich in straight chain paraffins.

4. A process according to any one of claims 1 to 3, **characterized in that** the feed comprises the C5 cut and isopentane from this cut is separated with the mono-branched paraffins.

5. A process according to any one of claims 1 to 3, **characterized in that** the feed comprises the C5 cut and isopentane from this cut is extracted from streams traversing the process using a deisopentaniser disposed upstream of one (or the) separation unit(s).

6. A process according to any one of claims 1 to 3, **characterized in that** the feed comprises the C5 cut and the isopentane from that cut is extracted from the streams traversing the process using a deisopentaniser disposed downstream of the separation units.

7. A process according to any one of claims 1 to 6, **characterized in that** it uses at least two separation units operating by adsorption.

8. A process according to any one of claims 1 to 6, **characterized in that** it uses at least one separation unit operating by adsorption and at least one permeation separation unit employing one or more membranes.

9. A process according to any one of claims 1 to 6, **characterized in that** it uses at least two permeation separation units employing one or more membranes.

10. A process according to any one of claims 1 to 9, **characterized in that** the feed originates from atmospheric distillation.

11. A process according to any one of claims 1 to 9, **characterized in that** the feed originates from a reforming unit.

12. A process according to claim 11, **characterized in that** the feed is a light reformate.

13. A process according to any one of claims 1 to 9, **characterized in that** the feed originates from a conversion unit.

14. A process according to claim 13, **characterized in that** the feed is a hydrocracking naphtha.
